# EUROPEAN PATENT SPECIFICATION

(11) **EP 2 153 780 B1**
(45) Date of publication and mention of the grant of the patent: **02.07.2014**
(21) Application number: 08014489.2
(22) Date of filing: 14.08.2008
(51) Int. Cl.: A61B 17/06

(54) **Carrier for surgical suture material connected to at least one needle**
Träger für chirurgisches, mit mindestens einer Nadel verbundenem Wundnahtmaterial
Support pour matériau de suture chirurgicale connecté à au moins une aiguille

(43) Date of publication of application: 17.02.2010
(73) Proprietor: B. Braun Surgical, S.A., 08191 Rubi (Barcelona) (ES)
(72) Inventor: Aranda Garcia, José Antonio, 08226 Terrassa (Barcelona) (ES); Afonso Sanmarti, Olga, 08202 Sabadell (ES)
(74) Representative: Patentanwälte Ruff, Wilhelm, Beier, Dauster & Partner

(56) References cited:
- US-A- 5 180 053
- US-A- 5 472 081
- US-A1- 2008 017 526

## Description

The invention relates to a carrier for surgical suture material connected to at least one needle, in the form of a reel body with a baseplate which on its rounded outer circumference possesses receptacles for the stitch material and at least one needle holder arranged on the baseplate and having a resilient tension element, a needle being capable of being tension-mounted between a tension element and a bearing serving as a counterbearing and connected immovably to the baseplate.

Carriers for suture material having a reel body are known. Reference may be made, in this context, to EP 1 475 046 A1 and to US 5,236,083 and EP 0 529 297 A2. A needle holder is described particularly in EP 0 529 297 A2. There, either one resilient tension element cooperates with a fixed stop or two tension elements are directed towards one another. The tension elements are designed as blocks which are integrally formed onto the baseplate and which project via a clearance provided in the baseplate. The blocks, in turn, possess a U-shaped recess which is open in the direction of the clearance of the baseplate. Owing to this recess, the front side, directed towards the other stop, of the respective block is resilient and can be pressed rearwards at its lower edge, thus giving rise to a spring action. The stop faces of the blocks may be of angular design or have a notch, with the result that a three-point mounting is obtained. The double stop obtained by virtue of the notch in this case comes to bear against the inside of a curved needle. The spring excursion of the tension elements is limited. Only highly specific needle sizes can consequently be used. Moreover, the spring force varies greatly with the spring excursion.

A needle park comprising a generally planar base and an elongated arm having a nib extending towards the base from a point near the free end of the arm is known from US 5, 180, 053. The elongated arm pivots in a plane which is perpendicular to the plane of the base. Document US 5,180,053 forms basis of claim 1.

The object on which the invention is based is to provide a carrier with needle holder, in which the spring force changes only a little with the deflection of the tension element and it is possible to hold needles of different thickness securely in the same way.

The invention is characterized in that the tension element is arranged parallel to the plane of the baseplate and is formed by a spring arm with two ends, one end of which is connected fixedly to the baseplate and the free end of which has at least one stop for the needles, the spring arm being pivotable in a pivoting plane parallel to the baseplate.

Since, in the invention, a spring arm is provided which is arranged parallel to the baseplate, the spring arm can be designed to be long, without this having an effect on the thickness of the carrier. Due to the length of the spring arm, the latter can be deflected over relatively long distances, without this having an adverse effect on the restoring force. This can be kept essentially constant over a relatively long spring excursion. In contrast to the prior art where the pivoting plane is arranged perpendicularly to the baseplate and the tension element lies in a plane which is essentially perpendicular to the baseplate in the state of rest, in the invention there is provision for the pivoting plane to be parallel to the baseplate, that is to say the spring arm moves in a plane parallel to the baseplate. Since a large amount of space is thus available, without the thickness of the carrier being impaired, it is possible to design the spring arm so as to be essentially rectilinear or curved only a little, curvatures of at most 45°, in particular at most 30°, being preferred.

Depending on the embodiment, the spring arm may be configured with a different length. As a rule, the length of the spring arm amounts to at least 1.5 times its width. Normally, the length is at least 3 times, preferably at least 8 times, as large. The cross section of the spring arm may be configured as desired. Normally, it is rectangular, the width being greater than the thickness. Lateral projections are preferred as stops for bearing against the needle. The stops of the stationary bearing and of the spring arm are preferably offset with respect to one another in the longitudinal direction of the spring arm. The stops are advantageously designed as stop faces. At least in the case of the spring arm, the stops are bevelled or the stop faces are configured as oblique faces, in such a way that the spacing between the stops is increased in the direction of the baseplate. What is achieved thereby is that the needles, in the tension-mounted state, are pressed in the direction of the baseplate.

The overall needle holder is preferably produced in one piece with the baseplate and preferably with the entire carrier by injection moulding. The baseplate advantageously has, in the region of the spring arms, clearances which correspond at least to The size of the spring arms. Plastics with elastic properties as material for the baseplate or the entire carrier are suitable Polyethylene and polypropylene are particularly preferred.

The needle holder is preferably a three-point mounting with a single and a double stop. As already mentioned, the stops are preferably offset with respect to one another in the longitudinal direction of the spring arm such that the single stop comes to lie between the stops of the double stop. There is advantageously provision, in the case of curved needles, for the double stop to come to bear on the outside of the curvature of the needles. The needles are held reliably as a result.

Advantageously provided on the baseplate, in the region of the free ends of the spring arms and, in particular, in the region of the needle tips, is a tongue which is capable of pivoting out of the plane of the baseplate and which engages at least partially under needles held in the holder. Thus, when the tongue pivots out, the needles can be erected in the holder. This makes removal easier.

In a particularly preferred embodiment of the invention, in the non-loaded operating state the spring arms are prestressed and bear under prestress against the bearing secured to the baseplate. Such prestressing contributes to bringing about a secure hold of the needles even when these are very thin.

The spring arms may run rectilinearly in the detensioned state and are then curved in the tensioned state. In another particularly preferred embodiment, there is provision for designing the springs such that they are curved in the detensioned state and are directed straight or run essentially rectilinearly in the tensioned state.

The prestress may be generated in that, during the production of the needle holder, the spring arms come to lie on the side facing away from the active side of the fixed stop and then, with the prestress being generated, can be lifted by the fixed stop and thereby brought into the operating state for receiving a needle. The excursion which the free end of the spring arm in this case overshoots amounts, as a rule, to 2 to 10 mm, in particular 4 to 6 mm. The spring excursion which is available to the spring arm, still in the non-loaded operating state, for deflection out of the fixed bearing, in order to form a gap for receiving the needle, advantageously amounts to at least 1 mm, preferably 2 to 3 mm. Consequently, needles of different thickness and even of different shape, for example also needles with a triangular cross section, can be received comfortably. Owing to the tensioning, the spring arm comes to lie at least partially outside the clearance assigned to it.

In one embodiment of the invention, a guide element is provided which, at least with the needle being tension-mounted, holds the at least one spring arm in its pivoting plane. This ensures a reliable tension-mounting of the needle. Preferably, the guide element has a portion which runs parallel to the baseplate and which engages over the spring arm at least when the needle is tension-mounted.

As a rule, at least two needle holders are provided on the baseplate. These are normally functionally independent of one another. They can be designed different or identically, particularly in terms of type and size. Normally, the at least two needle holders possess in each case stops separate from one another. The needle holders may be oriented next to one another such that at least two spring arms are arranged parallel to one another and are pivotable parallel to one another in the same direction. As a rule, the spring arms are designed to be of equal length. Identical force conditions are thereby afforded. There is advantageous provision, furthermore, for the single stop of the three-point mounting to be provided on the bearing connected fixedly to the baseplate and for the double stop to be provided on the spring arm.

Normally, two needle holders are sufficient on the carrier. Even more than two may be provided. Two or more threads, which are connected in each case to a needle, may be arranged on the carrier. It is also possible to provide a thread at each of its two ends with a needle. In the case of particularly large needles, it may be advantageous to provide only one needle holder on the baseplate. Where straight and relatively long needles are concerned, it may be advantageous, furthermore, to provide two needle holders which are offset rotationally symmetrically with respect to one another at 180°.

In another embodiment, two spring arms are designed resiliently in opposite directions. Here, each spring arm may be assigned a specific fixed bearing as a counterbearing. Advantageously, between the two spring arms, a common fixed bearing is located which has corresponding stops on its two outsides directed towards the spring arms. In the case of such a combined needle holder, two needles may likewise be mounted parallel to one another. In this instance, it is advantageous if only the spring arm which comes to bear against the outside of the curvature of one needle has a double stop, whereas the double stop for the other needle is provided on the fixed bearing. The associated spring arm, which then bears against the inside of the curvature of the second needle, then has only one stop.

Further features and advantages of the invention may be gathered from the following description of embodiments in conjunction with the drawing. In this case, the individual features may be implemented in each case in themselves or severally in one embodiment.

In the drawing:
- Figure 1: shows an embodiment of the carrier according to the invention holding a needle,
- Figure 2: shows another embodiment of the invention,
- Figure 3: shows a detail of the embodiment according to Figure 2,
- Figure 4: shows a further embodiment of the invention,
- Figure 5: shows yet another version, wherein only the portion of the carrier including the needle holder is illustrated, and
- Figure 6: shows a perspective of the needle holder of Figure 5.

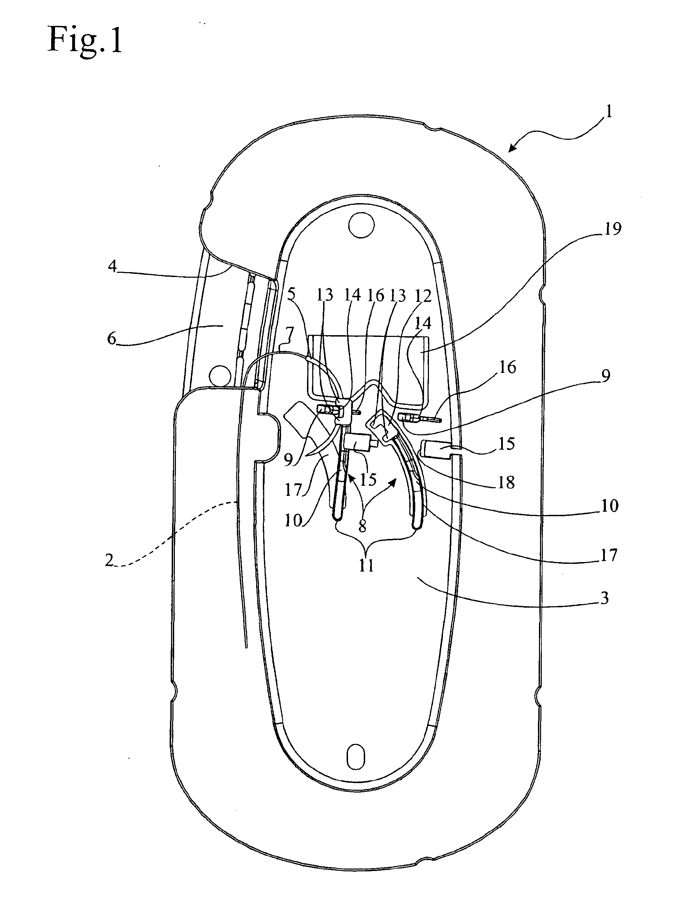

In the embodiment of the invention, as illustrated in Figure 1 of the drawing, a carrier 1 for a surgical suture 2 is provided, in the form of a reel body consisting of an elastic plastic with an approximately oval baseplate 3 which on the outer circumference has a groove-shaped channel 4 for receiving one or more wound threads 2 which are connected in each case to one or two needles 5. The groove-shaped channel 4 possesses a recess 6, through which the suture starts 7, which are connected to needles 5, may be led out and held in each case in a needle holder 8 in the region of the baseplate 3. In the present embodiment, two holders 8 are provided, one of them being provided with a curved needle 5, while the other still is in the unoccupied original state. However, the number may be larger or smaller. Each needle holder 8 is designed as a spring-loaded tension-mounting device, with a fixed bearing 9 formed in one piece on the baseplate 3 and projecting out of the plate and with an elongate spring arm 10 resilient against the fixed bearing 9. The spring arm is connected fixedly at one end 11 to the baseplate 3 and is formed in one piece with the latter. It possesses an approximately rectangular cross section, its thickness being smaller than its width. The thickness of the spring arm normally amounts to about 1.1 to 1.2 mm when the needle holder is designed particularly for very fine needles. Normally, the thickness of the spring arm amounts to approximately 1.5 to 1.6 mm. In the embodiment provided, the length of the spring arm 10 amounts to a multiple of the width, for example 10 to 15 times. The spring arm 10 is pivotable parallel to the plane of the baseplate 3, that is to say about an axis perpendicular to the baseplate, insofar as an axis can be referred to in the case of a plastic spring.

The spring arm 10 possesses at its free end 12 two stops 13 which are arranged so as to be offset with respect to one another in the longitudinal direction of the spring arm and which are directed towards the fixed bearing 9 in the operating state, the interspace between the two stops 13 at the free end 12 being such that a stop 14 provided on the fixed bearing 9 fits into the interspace between the stops 13. The spring arm 10, may in the still non-loaded operating state, have contact with the fixed bearing 9 and bear with prestress against the latter. The stops 13 and 14 provide a three-point mounting for a needle 5 to be tension-mounted, the stops 13 of the spring arm 10 or the double stop formed by these coming to bear against the outside of the curvature of the needle 5. The stops possess stop faces.

The stops 13 on the spring arm 10 are tapered in the direction of the baseplate 3 in that the bearing face of the stops is bevelled. Owing to the wedge action of the stops of the spring arm which is thereby brought about, a needle 5 held in a needle holder 8 is pressed in the direction of the baseplate 3. Furthermore, in the case of each needle holder, the baseplate 3 has provided on it a parallel guide 15 for the spring arms which engages at least partially over the respective spring arm 10, at least when a needle 5 is tension-mounted (left needle holder in Figure 1), and ensures that the elastic spring arm 10 remains in its pivoting plane and does not shift away from the baseplate 3.

The spring arm 10 possesses at its free end 12, in each case level with the two stops 13, also two sliding ribs which are directed towards the baseplate 3 and serve for a low-friction sliding of the free end 12 of the spring arm on the baseplate 3 and the longitudinal direction of which lies in the pivoting direction. Starting from the fixed stop 9, the baseplate 3 has a narrow rib 16 which is directed towards the spring arm 10 and which fits between the sliding ribs. The tension-mounted needle 5 lies on this rib 16. The rib holds the needle at a certain spacing from the surface of the baseplate 3 and consequently makes it easier to remove the needle, this being advantageous particularly in the case of very thin needles.

In the detensioned original state (right needle holder) which does not correspond to the operating state, the spring arms 10 lie on the other side of the fixed stop 9 and, for tensioning, are bent in each case over the fixed stops 9 and at the same time tensioned, so that they bear against the stop side of the stop 9.

At the locations at which the non-tensioned spring arms and the parallel guides are located, the baseplate 3 has correspondingly shaped clearances 17 and 18. These make it possible to produce the reel body 1 in a simple way, without complicated tools, by means of injection moulding.

In the embodiment illustrated, in the detensioned state, the spring arms possess a curvature which is generated during their production and which corresponds to an angle of about 30°. By the spring arm being prestressed, this curvature is largely cancelled, so that the spring arm is approximately rectilinear. On the other hand, it is also possible to design the spring arm so as to be rectilinear in the detensioned state, so that it is bent in the tensioned state.

Owing to the long free length (approximately 10 to 15 mm), the spring arms 10 possess a favourable spring characteristic with an essentially constant spring force, along with a long spring excursion. This makes it possible to tension-mount both very thin and thick needles 5, without the tension pressure being substantially different. The three-point mounting formed by the three stops 13 and 14 allows a secure fixing of the needles 5. Curved needles are preferably used such that the two stops 13 of the free end of the spring arm bear against the outside of the curvature of the needles 5.

Formed in the plane of the baseplate 3 by an essentially U-shaped clearance is a tongue 19 which is capable of being swung out of this plane and which is arranged in the prolongation of the spring arms 10 and is pivotable about a hinge remote from the spring arms. The rear needle parts projecting out of the needle holder can be erected by the tongue 19 being bent up, so that they can easily be removed from the holder by means of appropriate pincers.

The embodiment illustrated in Figure 1 possesses two needle holders 8 which are arranged parallel and next to one another. They are suitable particularly for thin to medium-thick needles, in particular for those which are curved.

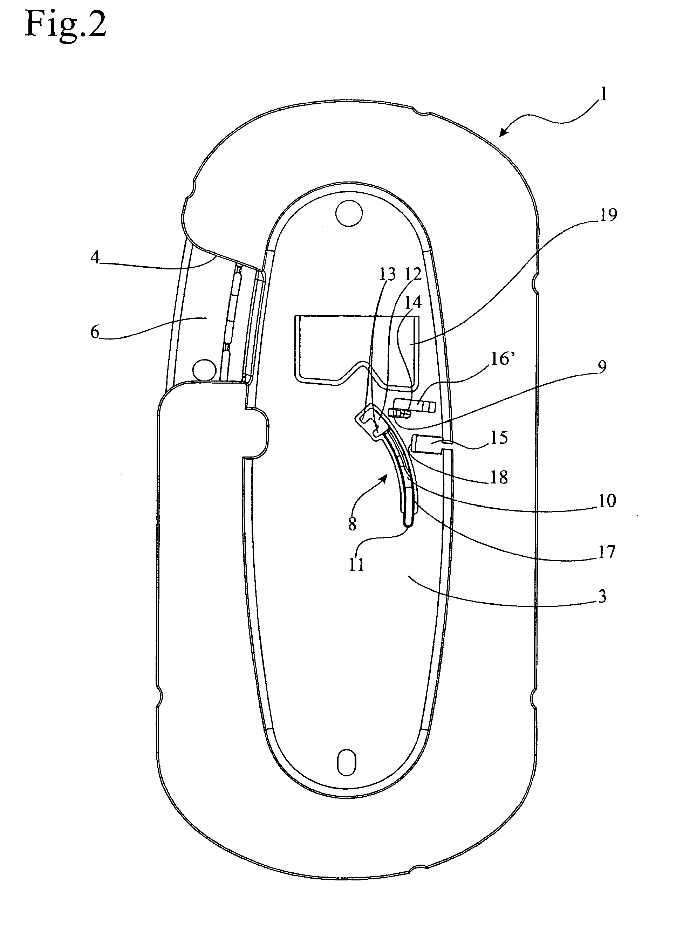

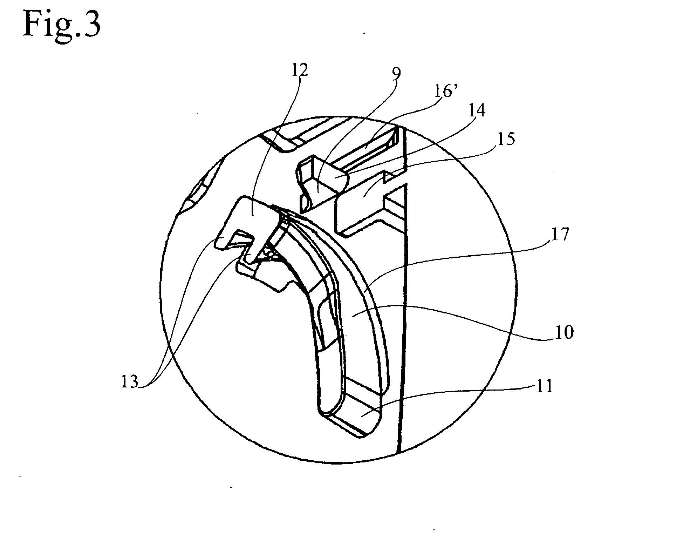

The embodiment according to Figures 2 and 3 possesses only one needle holder which is designed essentially identically to the embodiment according to Figure 1. The same reference symbols have therefore been used. It is suitable for particularly thick needles. Since thick needles can be grasped more easily than thin ones, in this embodiment the rib 16 on the fixed stop 9 may be omitted and be replaced by recess 16' in the base plate in which a sliding rip of the free end 12 of the spring arm 10 may engage. By this means it is avoided that the needle can slide under spring arm 10. In this version, the thickness of the spring arm 10 amounts, as a rule, to 1.5 to 1.6 mm. The length of the spring arm is the same as the length of the spring arm according to Figure 1.

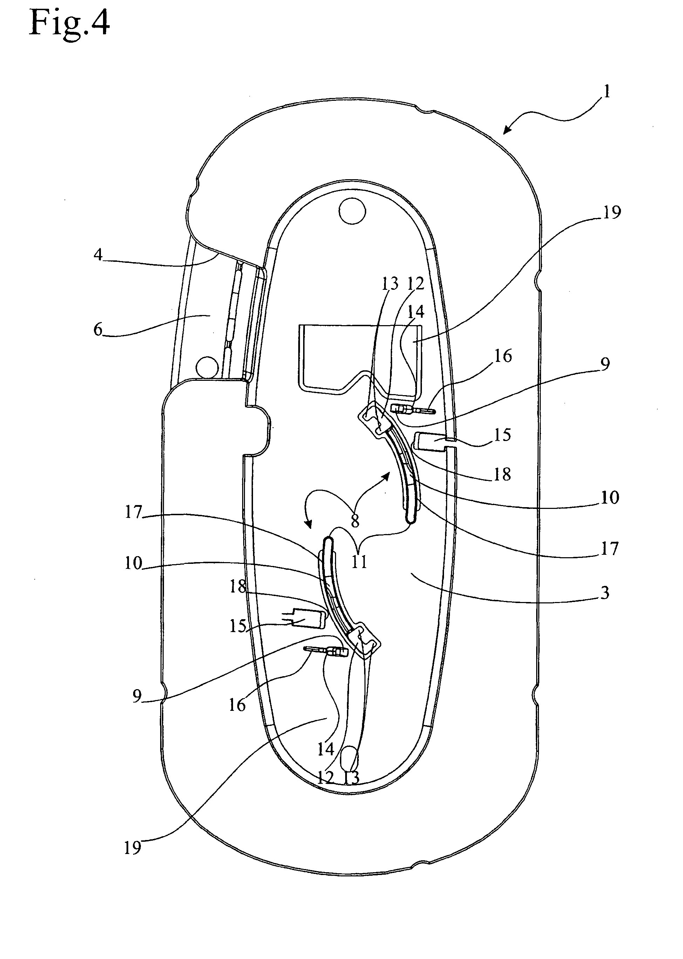

The embodiment according to Figure 4 corresponds essentially to the embodiment according to Figures 2 and 3, but two needle holders are provided, offset axially symmetrically at 180°, on the baseplate. This embodiment is suitable particularly for straight needles.

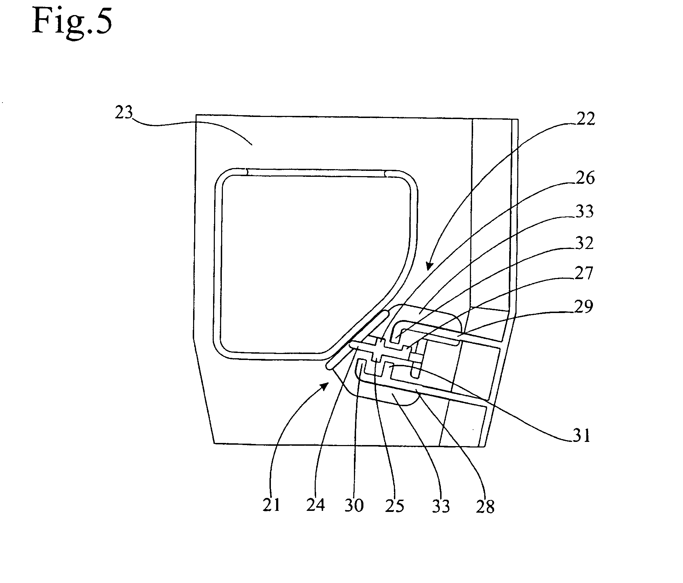

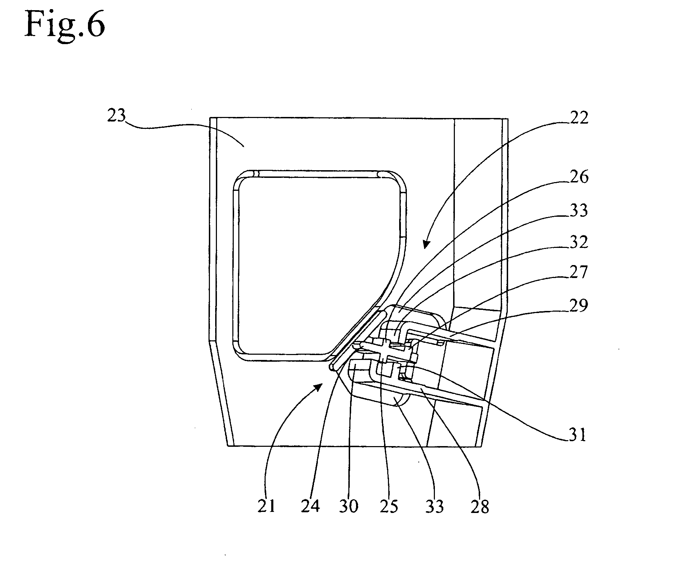

In the embodiment according to Figures 5 and 6, two needle holders 21 and 22 are combined with one another. A bearing 24 arranged fixedly on the baseplate 23 possesses stops 25, 26 and 27 on both sides. Spring arms 28 and 29 are provided on both sides of the bearing 24 and stand freely without prestress. They possess bevelled stops 30, 31 and 32. The two-sided stops of the bearing and of the free end of the spring arms engage one in the other, but do not touch one another. When a needle is inserted, the stops are pressed away from one another, with the result that the spring arms 28 and 29 are tensioned and the holding tension for the needles is generated. The free ends of the two spring arms 28 and 29 are displaced with respect to one another. A shorter spring arm 29 possesses only one stop 32 directed towards the fixed bearing 24. The fixed bearing, by contrast, possesses two stops 26 and 27 which are directed towards the shorter spring arm 29 and which are spaced apart from one another in the longitudinal direction to an extent such that they can receive between them the stop 32 of the shorter spring arm 29.

A longer spring arm 28 possesses at its free end two stops 30 and 31 which, in turn, are offset such that they can receive between them a stop 25, directed towards them, of the fixed bearing 24. Curved needles may again be mounted parallel to one another, in each case the two stops 26 and 27 of the fixed bearing 24 or the stops 30 and 31 of the longer spring arm coming to bear against the outside of the curvature of the needles.

The spring arms 28 and 29 and the fixed bearing 24 are arranged in a perforation 33 in the baseplate 23 and project with their longitudinal sides beyond the plate plane both on the operating side of the baseplate 23, on which the needles are tension-mounted, and on the rear side. As a result, the spring arms 28 and 29 are very wide and consequently also stable, so that short spring excursions are sufficient for generating the required spring force.

## Claims

1. Carrier for surgical suture material (2) connected to at least one needle (5), in the form of a reel body comprising
a baseplate (3; 23) which on its rounded outer circumference possesses receptacles (4) for the stitch material (2),
at least one needle holder (8; 21; 22) arranged on the baseplate and having a resilient tension element (10; 29; 30), wherein the tension element is formed by a spring arm (10; 28; 29) having two ends and being arranged parallel to the plane of the baseplate (3; 23) wherein one end (11) is connected fixedly to the baseplate (3),
the free end (12) of the spring arm comprises at least one stop (13; 30; 31; 32) for the needle(5),
**characterized in that** a bearing (9; 24) serving as a counterbearing is connected immovably to the baseplate (3; 23) and the spring arm (10; 28; 29) being pivotable in a pivoting plane parallel to the baseplate (3; 23) such that the needle can be tension-mounted between the tension element (10; 28; 29) and the counterbearing.

2. Carrier according to claim 1, **characterized in that** the spring arm (10; 28; 29) possesses a length which amounts at least to 1.5 times the width, preferably is at least 3 times and, in particular, at least 8 times as large as the width.

3. Carrier according to claim 1 or 2, **characterized in that** the spring arm (10; 28; 29) possesses an essentially rectangular cross section, and the stops (13; 14; 25; 26; 27; 30; 31; 32) are designed as lateral projections.

4. Carrier according to any of the preceding claims, **characterized in that** the stops (13; 14; 25; 26; 27; 30; 31; 32) have stop faces which come to bear against the needles.

5. Carrier according to any of the preceding claims, **characterized in that** stops (13; 30; 31; 32) provided, in particular stops on the spring arm, are bevelled and press the needles, in the tension-mounted state, in the direction of the baseplate.

6. Carrier according to any of the preceding claims, **characterized in that** the overall needle holder (8; 21; 22) is injection-moulded in one piece with the baseplate (3; 23), and the baseplate has in the region of the spring arms (10; 28; 29) clearances (17; 18; 33) which correspond at least to the size of the spring arms.

7. Carrier according to any of the preceding claims, **characterized in that** the needle holder (10; 28; 29) is a three-point mounting with a single stop (14; 25; 32) and with a double stop (13; 26; 27; 30; 31), the stops being offset with respect to one another in the longitudinal direction of the spring arm (10; 28; 29) such that the single stop (14; 25; 32) comes to lie between the stops (13; 26; 27; 30; 31) of the double stop.

8. Carrier according to claim 7, **characterized in that** in the case of curved needles (5), the double stop (13; 26; 27; 30; 31) comes to bear on the outside of the curvature of the needles.

9. Carrier according to any of the preceding claims, **characterized in that** provided on the baseplate (3), in the region of the free ends (12) of the spring arms (10), is a tongue (19) which is capable of pivoting out of the plane of the baseplate and which engages under needles (5) held in the holder (8) and, when pivoting out, lifts the needles (5) in the holder (8).

10. Carrier according to any of the preceding claims, **characterized in that** the at least one spring arm (10) is prestressed in the non-loaded operating state and bears with the prestress against the bearing (9) secured to the baseplate (3).

11. Carrier according to any of the preceding claims, **characterized in that** the at least one spring arm runs rectilinearly in the detensioned state and is curved in the tensioned state.

12. Carrier according to any of claims 1 to 10, **characterized in that** the at least one spring arm (10) is curved in the detensioned state and runs approximately rectilinearly in the tensioned state.

13. Carrier according to any of claims 6 to 12, **characterized in that**, in the tensioned state, the spring arm (10) lies at least partially outside the clearance (17), assigned to it, of the baseplate.

14. Carrier according to any of claims 10 to 13, **characterized in that**, in the non-loaded operating state, the at least one spring arm (10) is prestressed at the free end (12) over an arc length of 2 to 10 mm, in particular 4 to 6 mm.

15. Carrier according to any of the preceding claims, **characterized in that** the at least one spring arm (10; 28; 29) possesses a spring excursion between stops (13; 30; 31; 32) of the spring arm and stops (14; 25; 26; 27) of the bearing of at least 1 mm, preferably 2 to 3 mm.

16. Carrier according to any of the preceding claims, **characterized in that**, with a needle (5) tension-mounted, the at least one spring arm (10) is guided in its pivoting plane by at least one guide element (15).

17. Carrier according to claim 16, **characterized in that** the guide element (15) has a portion which runs parallel to the baseplate and which engages over the spring arm (10) when a needle (5) is tension-mounted.

18. Carrier according to any of the preceding claims, **characterized in that** at least two, in particular two needle holders (8; 21; 22), which are preferably designed identically, are provided on the baseplate (3; 23).

19. Carrier according to any of the preceding claims, **characterized in that** the at least two needle holders (8; 21; 22) have in each case separate stops (13; 14; 25; 26; 27; 30; 31; 32).

20. Carrier according to any of the preceding claims, **characterized in that** the at least two needle holders (8) possess spring arms (10) which are pivotable parallel to one another in the same direction.

21. Carrier according to any of the preceding claims, **characterized in that** the spring arms (10; 28; 29) of at least two needle holders (8; 21; 22) are of equal length.

22. Carrier according to any of claims 7 to 21, **characterized in that** the single stop (14) of the three-point mounting is provided on the bearing (9) connected fixedly to the baseplate and the double stop (13) is provided on the spring arm (10).

23. Carrier according to any of the preceding claims, **characterized in that** spring arms (28; 29) are designed resiliently in opposite directions.

24. Carrier according to claim 23, **characterized in that** a combined needle holder (21; 22) is provided, with two spring arms (28; 29) and with a common counterbearing (24) which is arranged between the spring arms and which is connected fixedly to the baseplate (23), and the counterbearing has on both sides stops (25; 26; 27) which are directed towards the stops of the spring arms.

## Patentansprüche

1. Träger für mit mindestens einer Nadel (5) verbundenes chirurgisches Nahtmaterial (2) in Form eines Spulenkörpers umfassend
eine Basisplatte (3; 23), die an ihrem abgerundeten Außenumfang Aufnahmen (4) für das Nahtmaterial (2) besitzt,
mindestens eine auf der Basisplatte angeordnete Nadelhalterung (8; 21; 22) mit einem federnden Spannelement (10; 29; 30), wobei das Spannelement von einem parallel zur Ebene der Basisplatte (3; 23) angeordneten Federarm (10; 28; 29) mit zwei Enden gebildet wird, dessen eines Ende (11) mit der Basisplatte (3) fest verbunden ist,
wobei das freie Ende (12) des Federarms mindestens einen Anschlag (13; 30; 31; 32) für die Nadel (5) aufweist,
**dadurch gekennzeichnet, dass**
ein als Gegenlager dienendes Lager (9; 24) mit der Basisplatte (3; 23) unbeweglich verbunden ist und der Federarm (10; 28; 29) in einer zur Basisplatte (3; 23) parallelen Schwenkebene derart schwenkbar ist, dass die Nadel zwischen dem Spannelement (10; 28; 29) und dem Gegenlager einspannbar ist.

2. Träger nach Anspruch 1, **dadurch gekennzeichnet, dass** der Federarm (10; 28; 29) eine Länge besitzt, die mindestens das 1,5-fache der Breite beträgt, vorzugsweise mindestens 3 mal, und insbesondere mindestens 8 mal, so groß ist wie die Breite.

3. Träger nach Anspruch 1 oder 2, **dadurch gekennzeichnet, dass** der Federarm (10; 28; 29) einen im Wesentlichen rechteckigen Querschnitt besitzt und die Anschläge (13; 14; 25; 26; 27; 30; 31; 32) als seitliche Vorsprünge ausgebildet sind.

4. Träger nach einem der vorhergehenden Ansprüche, **dadurch gekennzeichnet, dass** die Anschläge (13; 14; 25; 26; 27; 30; 31; 32) Anschlagflächen aufweisen, die zur Anlage an die Nadeln kommen.

5. Träger nach einem der vorhergehenden Ansprüche, **dadurch gekennzeichnet, dass** insbesondere am Federarm vorgesehene Anschläge (13; 30; 31; 32) abgeschrägt sind und die Nadeln im eingespannten Zustand in Richtung zur Basisplatte drücken.

6. Träger nach einem der vorhergehenden Ansprüche, **dadurch gekennzeichnet, dass** die gesamte Nadelhalterung (8; 21; 22) einstückig mit der Basisplatte (3; 23) gespritzt ist und die Basisplatte im Bereich der Federarme (10; 28; 29) Aussparungen (17; 18; 33) aufweist, die mindestens der Größe der Federarme entsprechen.

7. Träger nach einem der vorhergehenden Ansprüche, **dadurch gekennzeichnet, dass** die Nadelhalterung (10; 28; 29) eine Dreipunktlagerung mit einem einzelnen Anschlag (14; 25; 32) und einem Doppelanschlag (13; 26; 27; 30; 31) ist, wobei die Anschläge so gegeneinander in Längsrichtung des Federarms (10; 28; 29) versetzt sind, dass der einzelne Anschlag (14; 25; 32) zwischen den Anschlägen (13; 26; 27; 30; 31) des Doppelanschlages zu liegen kommt.

8. Träger nach Anspruch 7, **dadurch gekennzeichnet, dass** der Doppelanschlag (13; 26; 27; 30; 31) bei gekrümmten Nadeln (5) auf der Krümmungsaußenseite der Nadeln zur Anlage kommt.

9. Träger nach einem der vorhergehenden Ansprüche, **dadurch gekennzeichnet, dass** an der Basisplatte (3) im Bereich der freien Enden (12) der Federarme (10) eine aus der Ebene der Basisplatte ausschwenkbare Lasche (19) vorgesehen ist, die in der Halterung (8) gehaltene Nadeln (5) untergreift und beim Ausschwenken die Nadeln (5) in der Halterung (8) anhebt.

10. Träger nach einem der vorhergehenden Ansprüche, **dadurch gekennzeichnet, dass** der mindestens eine Federarm (10) im unbeladenen Betriebszustand vorgespannt ist und mit der Vorspannung an dem an der Basisplatte (3) festgelegten Lager (9) anliegt.

11. Träger nach einem der vorhergehenden Ansprüche, **dadurch gekennzeichnet, dass** der mindestens eine Federarm im entspannten Zustand geradlinig verläuft und im gespannten Zustand gekrümmt ist.

12. Träger nach einem der Ansprüche 1 bis 10, **dadurch gekennzeichnet, dass** der mindestens eine Federarm (10) im entspannten Zustand gekrümmt ist und im gespannten Zustand in etwa geradlinig verläuft.

13. Träger nach einem der Ansprüche 6 bis 12, **dadurch gekennzeichnet, dass** der Federarm (10) im gespannten Zustand mindestens teilweise außerhalb der ihm zugeordneten Aussparung (17) der Basisplatte liegt.

14. Träger nach einem der Ansprüche 10 bis 13, **dadurch gekennzeichnet, dass** der mindestens eine Federarm (10) im unbeladenen Betriebszustand am freien Ende (12) über eine Bogenlänge von 2 bis 10 mm, insbesondere 4 bis 6 mm, vorgespannt ist.

15. Träger nach einem der vorhergehenden Ansprüche, **dadurch gekennzeichnet, dass** der mindestens eine Federarm (10; 28; 29) einen Federweg zwischen Anschlägen (13; 30; 31; 32) des Federarmes und Anschlägen (14; 25; 26; 27) des Lagers von mindestens 1 mm, vorzugsweise 2 bis 3 mm, besitzt.

16. Träger nach einem der vorhergehenden Ansprüche, **dadurch gekennzeichnet, dass** der mindestens eine Federarm (10) bei eingespannter Nadel (5) durch mindestens ein Führungselement (15) in seiner Schwenkebene geführt ist.

17. Träger nach Anspruch 16, **dadurch gekennzeichnet, dass** das Führungselement (15) einen parallel zur Basisplatte verlaufenden Abschnitt aufweist, der den Federarm (10) bei eingespannter Nadel (5) übergreift.

18. Träger nach einem der vorhergehenden Ansprüche, **dadurch gekennzeichnet, dass** an der Basisplatte (3; 23) mindestens zwei, insbesondere zwei, Nadelhalterungen (8; 21; 22) vorgesehen sind, die vorzugsweise gleich ausgebildet sind.

19. Träger nach einem der vorhergehenden Ansprüche, **dadurch gekennzeichnet, dass** die mindestens zwei Nadelhalterungen (8; 21; 22) jeweils getrennte Anschläge (13; 14; 25; 26; 27; 30; 31; 32) aufweisen.

20. Träger nach einem der vorhergehenden Ansprüche, **dadurch gekennzeichnet, dass** die mindestens zwei Nadelhalterungen (8) Federarme (10) besitzen, die parallel zueinander in die gleiche Richtung schwenkbar sind.

21. Träger nach einem der vorhergehenden Ansprüche, **dadurch gekennzeichnet, dass** die Federarme (10; 28; 29) der mindestens zwei Nadelhalterungen (8; 21; 22) gleich lang sind.

22. Träger nach einem der Ansprüche 7 bis 21, **dadurch gekennzeichnet, dass** der einzelne Anschlag (14) der Dreipunktlagerung an dem mit der Basisplatte fest verbundenen Lager (9) vorgesehen ist und der Doppelanschlag (13) am Federarm (10) vorgesehen ist.

23. Träger nach einem der vorhergehenden Ansprüche, **dadurch gekennzeichnet, dass** die Federarme (28; 29) in entgegengesetzter Richtung federnd ausgebildet sind.

24. Träger nach Anspruch 23, **dadurch gekennzeichnet, dass** eine kombinierte Nadelhalterung (21; 22) vorgesehen ist mit zwei Federarmen (28; 29) und einem gemeinsamen zwischen den Federarmen angeordneten Gegenlager (24), das mit der Basisplatte (23) fest verbunden ist, und das Gegenlager auf beiden Seiten Anschläge (25; 26; 27) aufweist, die den Anschlägen der Federarme zugerichtet sind.

## Revendications

1. Support pour matériau (2) de suture chirurgicale relié à au moins une aiguille (5), sous forme de corps de dévidoir comportant
une plaque de base (3 ; 23) qui, sur sa circonférence externe arrondie, possède des réceptacles (4) destinés au matériau (2) pour les points,
au moins un porte-aiguille (8 ; 21 ; 22) disposé sur la plaque de base et muni d'un élément (10 ; 29 ; 30) de tension résilient, dans lequel l'élément de tension est formé par un bras (10 ; 28 ; 29) à ressort présentant deux extrémités et étant disposé parallèlement au plan de la plaque de base (3 ; 23), une extrémité (11) étant reliée de manière fixe à la plaque de base (3),
l'extrémité (12) libre du bras à ressort comporte au moins une butée (13 ; 30 ; 31 ; 32) pour l'aiguille (5),
**caractérisé en ce qu'**une portée (9 ; 24) servant de contre-portée est reliée de manière inamovible à la plaque de base (3 ; 23) et le bras (10 ; 28 ; 29) à ressort est apte à pivoter dans un plan de pivotement parallèle à la plaque de base (3 ; 23) de sorte que l'aiguille peut être montée sous tension entre l'élément (10 ; 28 ; 29) de tension et la contre-portée.

2. Support selon la revendication 1, **caractérisé en ce que** le bras (10 ; 28 ; 29) à ressort possède une longueur qui s'élève à au moins 1,5 fois la largeur, de préférence au moins 3 fois et, en particulier, au moins 8 fois aussi grande que la largeur.

3. Support selon la revendication 1 ou 2, **caractérisé en ce que** le bras (10 ; 28 ; 29) à ressort possède une section transversale essentiellement rectangulaire, et les butées (13 ; 14 ; 25 ; 26 ; 27 ; 30 ; 31 ; 32) sont conçues comme saillies latérales.

4. Support selon l'une quelconque des revendications précédentes, **caractérisé en ce que** les butées (13 ; 14 ; 25 ; 26 ; 27 ; 30 ; 31 ; 32) présentent des faces de butée qui viennent porter contre les aiguilles.

5. Support selon l'une quelconque des revendications précédentes, **caractérisé en ce que** les butées (13 ; 30 ; 31 ; 32) fournies, en particulier les butées sur le bras à ressort, sont biseautées et appuient sur les aiguilles, dans l'état monté sous tension, dans la direction de la plaque de base.

6. Support selon l'une quelconque des revendications précédentes, **caractérisé en ce que** l'ensemble du porte-aiguille (8 ; 21 ; 22) est moulé par injection en une seule pièce avec la plaque de base (3 ; 23), et la plaque de base présente, dans la zone des bras (10 ; 28 ; 29) à ressort, des dégagements (17 ; 18 ; 33) qui correspondent au moins à la taille des bras à ressort.

7. Support selon l'une quelconque des revendications précédentes, **caractérisé en ce que** le porte-aiguille (10 ; 28 ; 29) est un montage à trois points avec une simple butée (14 ; 25 ; 32) et avec une double butée (13 ; 26 ; 27 ; 30 ; 31), les butées étant décalées les unes par rapport aux autres dans la direction longitudinale du bras (10 ; 28 ; 29) à ressort de sorte que la simple butée (14 ; 25 ; 32) vient reposer entre les butées (13 ; 26 ; 27 ; 30 ; 31) de la double butée.

8. Support selon la revendication 7, **caractérisé en ce que** dans le cas d'aiguilles (5) courbes, la double butée (13 ; 26 ; 27 ; 30 ; 31) vient porter sur l'extérieur de la courbure des aiguilles.

9. Support selon l'une quelconque des revendications précédentes, **caractérisé en ce que** sur la plaque de base (3), dans la zone des extrémités (12) libres des bras (10) à ressort, une languette (19) est fournie qui est apte à pivoter à distance du plan de la plaque de base et se met en prise sous les aiguilles (5) retenues dans le porte-aiguille (8) et qui, lorsqu'elle pivote à distance de cette dernière, soulève les aiguilles (5) dans le porte-aiguille (8).

10. Support selon l'une quelconque des revendications précédentes, **caractérisé en ce que** ledit au moins un bras (10) à ressort est précontraint dans l'état de fonctionnement non chargé et porte avec la précontrainte contre la portée (9) fixée à la plaque de base (3).

11. Support selon l'une quelconque des revendications précédentes, **caractérisé en ce que** ledit au moins un bras à ressort s'étend de manière rectiligne dans l'état non tendu et est courbé dans l'état tendu.

12. Support selon l'une quelconque des revendications 1 à 10, **caractérisé en ce que** ledit au moins un bras (10) à ressort est courbe dans l'état non tendu et s'étend à peu près de manière rectiligne dans l'état tendu.

13. Support selon l'une quelconque des revendications 6 à 12, **caractérisé en ce que**, dans un état tendu, le bras (10) à ressort repose au moins partiellement à l'extérieur du dégagement (17), qui lui est attribué, de la plaque de base.

14. Support selon l'une quelconque des revendications 10 à 13, **caractérisé en ce que**, dans l'état de fonctionnement non chargé, ledit au moins un bras (10) à ressort est précontraint au niveau de l'extrémité (12) libre sur une longueur d'arc de 2 à 10 mm, en particulier de 4 à 6 mm.

15. Support selon l'une quelconque des revendications précédentes, **caractérisé en ce que** ledit au moins un bras (10 ; 28 ; 29) à ressort possède une course de ressort entre les butées (13 ; 30 ; 31 ; 32) du bras à ressort et les butées (14 ; 25 ; 26 ; 27) de la portée d'au moins 1 mm, de préférence de 2 à 3 mm.

16. Support selon l'une quelconque des revendications précédentes, **caractérisé en ce que**, avec une aiguille (5) montée sous tension, ledit au moins un bras (10) à ressort est guidé dans son plan de pivotement par au moins un élément (15) guide.

17. Support selon la revendication 16, **caractérisé en ce que** l'élément (15) guide présente une partie qui s'étend parallèlement à la plaque de base et se met en prise sur le bras (10) à ressort lorsqu'une aiguille (5) est montée sous tension.

18. Support selon l'une quelconque des revendications précédentes, **caractérisé en ce qu'**au moins deux, en particulier deux porte-aiguille (8 ; 21 ; 22), de préférence conçus de manière identique, sont fournis sur la plaque de base (3 ; 23).

19. Support selon l'une quelconque des revendications précédentes, **caractérisé en ce que** lesdits au moins deux porte-aiguille (8 ; 21 ; 22) ont dans chaque cas des butées (13 ; 14 ; 25 ; 26 ; 27 ; 30 ; 31 ; 32) séparées.

20. Support selon l'une quelconque des revendications précédentes, **caractérisé en ce que** lesdits au moins deux porte-aiguille (8) possèdent des bras (10) à ressort aptes à pivoter parallèlement les uns par rapport aux autres dans la même direction.

21. Support selon l'une quelconque des revendications précédentes, **caractérisé en ce que** les bras (10 ; 28 ; 29) à ressort d'au moins deux porte-aiguille (8 ; 21 ; 22) sont de même longueur.

22. Support selon l'une quelconque des revendications 7 à 21, **caractérisé en ce que** la simple butée (14) du montage à trois points est fournie sur la portée (9) reliée de manière fixe à la plaque de base et la double butée (13) est fournie sur le bras (10) à ressort.

23. Support selon l'une quelconque des revendications précédentes, **caractérisé en ce que** les bras (28 ; 29) à ressort sont conçus de manière résiliente dans des directions opposées.

24. Support selon la revendication 23, **caractérisé en ce qu'**un porte-aiguille (21 ; 22) combiné est fourni, avec deux bras (28 ; 29) à ressort et avec une contre-portée (24) commune qui est disposée entre les bras à ressort et qui est relié de manière fixe à la plaque de base (23), et la contre-portée présente sur ses deux côtés des butées (25 ; 26 ; 27) qui sont dirigées vers les butées des bras à ressort.
